# FASCICULE DE BREVET EUROPEEN

(11) **EP 2 135 886 B1**
(45) Date de publication et mention de la délivrance du brevet: **30.05.2012**
(21) Numéro de dépôt: 09305557.2
(22) Date de dépôt: 17.06.2009
(51) Int. Cl.: C08G 63/06, C08G 63/82, C08G 63/91

(54) **Résines d'origine naturelle issues d'huile végétale et d'hydroxy acides**
Harze aus natürlicher Quelle, hergestellte aus Pflanzenöl und Hydroxycarbonsäuren
Resins from natural source based on vegetable oil and hydroxyacids

(30) Priorité: 20.06.2008 FR 0854108
(43) Date de publication de la demande: 23.12.2009
(73) Titulaire: COLAS, 92100 Boulogne-Billancourt (FR); VALAGRO, 86022 Poitiers (FR)
(72) Inventeur: Deneuvillers, Christine, 78310, MAUREPAS (FR); Piccirilli, Antoine, 86000, POITIERS (FR)
(74) Mandataire: Michelet, Alain

(56) Documents cités:
- EP-A- 0 990 672
- EP-A- 1 367 080
- US-A- 2 929 827
- US-A- 5 371 176
- US-A1- 2005 026 999
- US-A1- 2006 183 815
- US-A1- 2006 243 669

## Description

L'invention concerne des résines organiques dérivées d'une huile ou d'une graisse d'origine naturelle. L'invention concerne également le procédé de préparation de ces résines. Cette invention concerne des bio-produits obtenus à partir de ressources renouvelables.

On entend par « bioproduit », un produit fabriqué à partir de matières premières renouvelables par opposition aux matières premières d'origine fossile comme le pétrole. Les bioproduits peuvent remplacer ou améliorer d'autres produits composés d'éléments non renouvelables. Les bioproduits se retrouvent dans tous les secteurs : plastiques (emballage alimentaire), textiles (vêtements et fibres diverses), détergents et hygiènes (produits ménagers et d'hygiène corporelle), encres (encres d'imprimerie), cosmétiques...

Ces bioproduits présentent de nombreux avantages mais surtout un intérêt environnemental. D'une part, ces produits peuvent se substituer aux matières premières d'origine fossile. Les ressources pétrolières sont donc préservées. D'autre part, les biopolymères sont en général plus facilement dégradés ce qui n'est pas le cas des molécules constituant la plupart des plastiques à base de pétrole. En outre, l'utilisation de ces produits permet de réduire les gaz à effet de serre.

Il existe déjà sur le marché des résines à base de produit d'origine naturelle. On peut citer notamment les huiles végétales qui ont été utilisées comme matière première pour la fabrication de résines alkydes semi-naturelles (polyesters plastifiés). Ces résines sont obtenues en condensant des huiles végétales avec des anhydrides d'origine pétrochimique ou synthétique tels que les anhydrides maléique et phtalique (A. Karleskind, Manuel des Corps Gras, pp 1461 - 1465).

Des résines plastiques ont également été synthétisées à partir d'huiles végétales et de monomères tels que le styrène, le cyclopentadiène et le divinylbenzène, composés issus de la pétrochimie, selon un procédé de polymérisation cationique.

On connaît également des bio-polymères synthétisés en faisant réagir l'huile de soja époxydée avec l'acide acrylique ou avec l'anhydride maléique en présence ou non de polyols synthétiques tels que le néopentyl glycol (NPG).

Cependant, toutes ces résines ne comportent pas exclusivement des matières premières d'origine naturelle.

Des travaux de recherche ont été menés pour développer de nouveaux matériaux plastiques d'origine essentiellement naturelle.

Ces biopolymères obtenus à partir de matières premières renouvelables sont en général des polymères naturellement présents dans les organismes vivants ou synthétisés par ces derniers à partir de ressources renouvelables. Ils peuvent donc être :
- naturels (extraits de végétaux traités),
- d'origine microbienne,
- synthétisés par des organismes vivants, ou
- d'origine animale.

Ces biopolymères sont par exemple réalisés à partir de glucides, de lipides, de protéines et de polyphénols extraits des végétaux, et notamment de cellulose, d'amidon, de sucres, d'huiles végétales ou animales, de protéines végétales ou animales (HN Rabetafika et coll, « Les Polymères Issus du Végétal : Matériaux à Propriétés spécifiques pour des Applications Ciblées en Industries Plastique », Biotechnol. Agron. Soc. Environn. 2006, 10 (3), pp 185-196).

Parmi ces produits, on peut citer notamment l'acide polylactique (PLA) obtenu à partir du maïs.

La demande de brevet US 2005/0026999 divulgue des compositions comprenant des monoglycérides et/ou des diglycérides d'acide gras estérifiés par d'acide lactique. Ce document D1 ne divulgue pas un composé pouvant comprendre une chaîne résultant de la condensation de 6 unités d'hydroxy acide.

La demande US 2006/243669 divulgue des compositions « décontaminantes » pour produits chimiques aqueux. Ces compositions comprennent des esters d'acides gras et de polyols comprenant de l'acide lactique. Les exemples divulguent des composés obtenus par réaction d'acide lactique, d'acide gras et de glycérol. Il est précisé que ces composés peuvent comprendre des chaînes de 3 à 9 unités « acide lactique ». Ce document ne divulgue pas non plus un procédé comprenant une étape de mise en réaction d'au moins un hydroxy acide ou un ester d'hydroxy acide en excès, ou d'un poly(hydroxy acide) déjà formé, et d'un mono et/ou un diglycéride.

La demande EP 0990 672 divulgue des oligomères biodégradables de polyester produits à partir d'hydroxy acide et de glycérol. Ces composés ne sont pas des esters d'acide gras.

On peut également citer la demande de brevet EP 1 367 080 qui divulgue des polymères ramifiés à partir d'acide lactique et de glycérine ou d'autres polyols végétaux.

Il existe toutefois un besoin de développer d'autres biopolymères d'origine naturelle, notamment des résines à base de matières premières renouvelables, susceptibles de se substituer dans diverses applications aux résines pétrochimiques, synthétiques ou semi-naturelles, traditionnellement utilisées.

Ces biopolymères doivent donc pouvoir se substituer aux produits composés d'éléments non renouvelables tels que les matières premières d'origine fossile, être biofragmentables, biodégradables et de faible écotoxicité. Ces produits doivent en outre être réalisés de préférence à partir de matières premières naturelles n'ayant pas d'équivalent de synthèse à un coût abordable. Parmi ces composés, les résines naturelles présentant des propriétés thermoplastiques sont particulièrement recherchées.

On entend par « matière première et composé d'origine naturelle », tout produit extrait de la biomasse renouvelable, terrestre et marine, ou d'organismes vivants (animaux, micro-organismes), ou encore obtenus suite à l'action de micro-organismes (ex.enzymes, bactéries) sur ces composés et matières premières naturelles selon des procédés de bio-fermentation ou de bio-synthèse.

On entend par « thermoplastique », une matière plastique qui fond sous l'action de la chaleur ou, tout au moins, se ramollit suffisamment pour pouvoir être mise en forme un nombre infini de fois, sans modification de ses propriétés. Plus particulièrement, on entend par « propriété ou comportement thermoplastique » au sens de l'invention, une résine dont la viscosité diminue avec l'augmentation de la température, ce qui permet une manipulation aisée à une température élevée modérée, et qui recouvrent ses propriétés mécaniques aux températures d'usage.

Le demandeur a découvert des nouvelles résines d'origine exclusivement végétale présentant des propriétés thermoplastiques avantageuses permettant d'utiliser ces résines dans diverses applications.

L'invention concerne donc des résines organiques dérivées d'une huile ou d'une graisse d'origine naturelle comprenant des monoglycérides et/ou des diglycérides, estérifiés par un poly(hydroxy acide) répondant à la formule suivante : 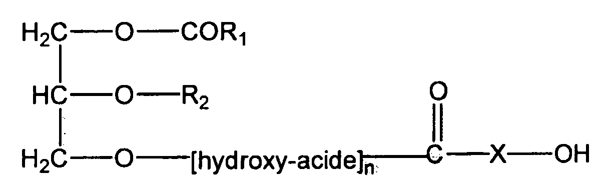 dans laquelle,
- R₁ est une chaîne hydrocarbonée aliphatique comportant 6 à 32 atomes de carbone, saturée ou insaturée, éventuellement substituée par des groupements alkyles ou hydroxyles,
- R₂ est un atome d'hydrogène, un groupe -COR₄ avec R₄ défini de la même façon que R₁ ou un groupe estérifié de poly(hydroxy acide),
- le groupement poly(hydroxy acide) correspondant à [hydroxy acide]ₙ-CO-X-OH est une chaîne linéaire ou ramifiée, obtenue par condensation de monomères d'hydroxy acide semblables ou différents,
   - en fonction de la nature de l'hydroxy acide, X = -CH₂, -CHR, R étant un groupe alkyle de 1 à 5 atomes de carbone et comportant 0 à 5 fonction(s) hydroxyle,
   - n représente le nombre de motifs d'hydroxy acide identiques ou différents et est compris entre 10 et 2000.

La résine selon l'invention présente en outre les caractéristiques suivantes seules ou en combinaison :
- les hydroxy acides sont choisis parmi les acides α-hydroxylés tels que l'acide lactique, l'acide citrique, l'acide malique, l'acide tartarique, l'acide ascorbique et les acides β-hydroxylés tels que l'acide glycolique, l'acide salicylique, l'acide β-hydroxy butyrique, de préférence parmi l'acide lactique, l'acide citrique et l'acide malique,
- le nombre de motifs d'hydroxy acide identiques ou différents n est compris entre 5 et 1000, de préférence compris entre 5 et 500, et mieux encore 5 et 120,
- le nombre de motifs d'hydroxy acide identiques ou différents n est compris entre 10 et 1000, de préférence compris entre 10 et 500, et mieux encore 10 et 120,
- les monoglycérides et/ou diglycérides sont obtenus à partir d'huiles végétales ou animales choisies parmi les huiles de colza oléique et érucique, l'huile de lin, l'huile de tournesol, l'huile de ricin, l'huile de pourghère (*Jatropha curcas*), l'huile de soja, l'huile de palme, l'huile de palmiste, l'huile de coprah, l'huile de maïs, l'huile de coton, l'huile d'arachide, l'huile de son de riz, l'huile d'olive, l'huile de tung, les huiles de poisson, de micro et macro-algues, l'huile de suif et de tall-oil,
- l'huile végétale comprend des acides gras comprenant entre 12 et 20 atomes de carbone et de préférence des acides gras en C18,
- la résine est essentiellement d'origine naturelle, de préférence d'origine végétale.

L'invention concerne également un procédé de préparation d'une résine organique dérivée d'une huile ou d'une graisse d'origine naturelle tel que défini dans les revendications 7 à 15. Ce procédé comporte une étape (b) de mise en réaction :
- d'au moins un hydroxy acide ou d'un ester d'hydroxy acide en excès, ou d'un poly(hydroxy acide) déjà formé, et
- d'un mono et/ou un diglycéride.

Le monoglycéride et/ou le diglycéride a été obtenu au préalable lors d'une étape (a) soit :
- par glycérolyse des triglycérides, ou
- par estérification du glycérol par les acides gras.

Le procédé de préparation selon l'invention présente en outre les caractéristiques suivantes seule ou en combinaison :
- l'étape (b) est réalisée par réaction de 1 à 30 % en poids, de préférence 1 à 20 % en poids de monoglycéride et/ou de diglycéride avec 70 à 99 % en poids, de préférence 80 à 98 % en poids par rapport au poids total de la résine d'hydroxy acide ou d'un poly(hydroxy acide) déjà formé,
- on utilise dans l'étape (b) un catalyseur choisi dans le groupe constitué par les sels organiques d'étain, de fer, de zinc, d'aluminium, les acides minéraux ou organiques, les catalyseurs basiques, de préférence, le catalyseur est l'éthylhexanoate d'étain (SnOct₂),
- l'étape (b) est réalisée avec un rapport molaire [hydroxy-acide]/[nombre de fonctions acide + hydroxyle] compris entre 6 et 1000, de préférence compris entre 6 et 500, et mieux encore entre 6 et 120,
- l'étape (b) dure de 5 et 12 heures, de préférence environ 9 heures

Les résines selon l'invention sont dérivées d'une huile ou d'une graisse d'origine naturelle en ce sens qu'elles sont obtenues à partir de monoglycéride ou de diglycéride. Ces monoglycérides et diglycérides sont eux mêmes obtenus à partir de triglycéride qui est le constituant principal des huiles végétales et des graisses animales.

En effet, les huiles végétales sont des huiles à forte teneur en triglycérides ou essentiellement constituées de triglycérides d'esters d'acide(s) gras et de glycérol dont les acides gras peuvent être saturés ou insaturés, linéaires ou ramifiés, comportant de 6 à 32 atomes de carbone et éventuellement 0 à 10 insaturations et 0 à 5 fonctions hydroxyle (-OH).

Parmi les huiles végétales convenant à l'invention, on peut citer les huiles de colza oléique et érucique, l'huile de lin, l'huile de tournesol, l'huile de ricin, l'huile de soja, l'huile de palme, l'huile de palmiste, l'huile de coprah, l'huile de maïs, l'huile de coton, l'huile d'arachide, l'huile de son de riz, l'huile d'olive, l'huile de tung, l'huile de pourghère (*Jatropha curcas*). L'huile de pourghère extraites des graines mûres de *Jatropha curcas* est une huile liquide à température ambiante, de type insaturé et avec une prédominance des acides gras oléique (43-53 %), linoléique (20-32 %) et palmitique (13-15 %).

D'autres sources de triglycérides naturels sont aussi utilisables telles que les huiles de poisson, de micro et macro-algues, de suif et de tall-oil.

De façon préférée, on choisira plutôt les huiles présentant des acides gras comportant entre 12 et 20 atomes de carbone et plus idéalement riches en C18 tels que l'acide oléique, linoléique ou linolénique.

Les hydroxy acides sont des acides organiques qui se définissent par la présence d'au moins une fonction hydroxyle (-OH) et d'au moins une fonction carboxylique (-COOH). Les hydroxy-acides naturels selon l'invention peuvent comporter 1 à 5 fonctions acides et 1 à 6 fonctions hydroxyles situées en position alpha, béta, gamma et delta de la fonction acide. Les α-hydroxyacides portent la fonction hydroxyle sur le carbone adjacent à la fonction acide carboxylique (soit en position 1 de la fonction acide), alors que les β-hydroxyacides portent la fonction hydroxyle sur le deuxième carbone adjacent à la fonction acide carboxylique (soit en position 2 de la fonction acide).

Parmi les hydroxy acides naturels convenant à l'invention; on peut citer l'acide lactique (sous forme D, L et racémique), l'acide citrique, l'acide malique, l'acide tartrique, l'acide glycolique, l'acide salicylique et l'acide β-hydroxybutyrique. On utilise de préférence l'acide lactique, citrique et malique. Les polyhydroxyacides greffés appartiennent donc au groupe constitué par le polylactate, le polymalate, le polyglycolate, le polycitrate issus de la condensation des hydroxy acides naturels correspondant.

La masse molaire moyenne d'une chaîne de poly(hydroxy acide) estérifiée correspondant au groupement [hydroxy acide]ₙ-CO-X-OH est de préférence comprise entre 350 et 100 000 g.mol⁻¹, de préférence entre 350 et 20 000 g.mol⁻¹.

Les résines selon l'invention sont donc essentiellement d'origine naturelle car elles sont obtenues à partir de dérivés d'une huile ou d'une graisse d'origine naturelle et d'hydroxy acides naturels. Selon l'invention, on entend par « essentiellement d'origine naturelle », une résine qui comporte, par rapport au poids total de la résine, au moins 95%, de préférence au moins 99% et mieux encore 100% en poids de composés d'origine naturelle.

Le procédé de préparation des résines selon l'invention incluant l'étape préalable d'obtention de monoglycérides ou de diglycérides peut être schématisé de la façon suivante.
Etape 1 : Préparation d'un mélange de monoglycérides et de diglycérides : 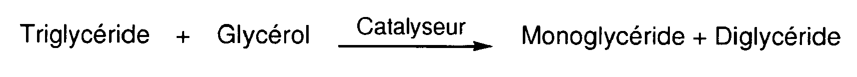
Etape 2 : Estérification des monoglycérides et/ou des diglycérides par :
   a) un hydroxy-acide en excès : 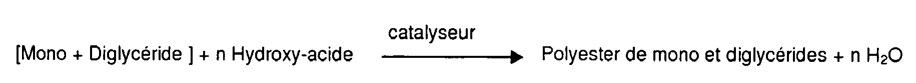
   b) un polyhydroxyacide déjà formé

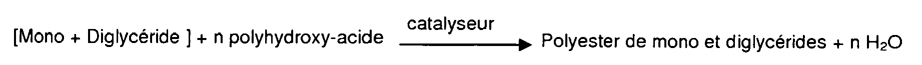

Les résines selon l'invention correspondent donc soit :
- à des monoglycérides mono-estérifiés par un hydroxy acide polymérisé,
- à des monoglycérides di-estérifiés par un hydroxy acide polymérisé, ou
- à des diglycérides estérifiés par un hydroxy acide polymérisé.

Lorsque que le monoglycéride et/ou le diglycéride est obtenu par glycérolyse des triglycérides, le rapport molaire glycérol/huile est compris entre 0,5 et 5. Pour obtenir un mélange riche en diglycéride, on choisit un rapport molaire glycérol/huile compris entre 0,9 et 1,1. Pour obtenir un mélange riche en monoglycéride, on choisit un rapport molaire glycérol/huile compris entre 1,9 et 2,1.

Le glycérol utilisé est de préférence d'origine végétale ou animale.

Le catalyseur utilisé dans l'étape (a) d'obtention des mono et diglycérides par glycérolyse peut être choisi dans le groupe des catalyseurs basiques homogènes et hétérogènes : NaOH, KOH, CaO, BaO, LiOH, Na₂CO₃, K₂CO₃, les oxydes de terre rares, les perovskytes, ZnO, ZnCl₂, SnCl₂, le stéarate de lithium. Des catalyseurs acides de transestérification peuvent aussi être utilisés tels que les résines acides, les zéolithes, les alumines, HCl, H₂SO₄, l'acide para-toluène sulfonique. De préférence, on choisira le catalyseur basique NaOH.

Cette étape est réalisée à une température comprise entre 60 et 280°C, de préférence entre 210 et 230°C.

Selon un autre mode de réalisation de l'invention, les monoglycérides et diglycérides peuvent être fabriqués selon un procédé d'estérification du glycérol par les acides gras selon l'art connu de l'homme de métier.

Le catalyseur de l'étape (b) permettant d'activer la réaction d'estérification des monoglycérides et diglycérides ainsi que la condensation de l'hydroxy acide sur lui-même conduisant au polyester, est de préférence choisi dans le groupe constitué par des sels organiques de Sn, Fe, Zn, Al, des acides minéraux ou organiques, des catalyseurs basiques. De préférence, le catalyseur est l'éthylhexanoate d'étain (SnOct2).

Le rapport molaire [hydroxy acide]/[nombre de fonctions acides + hydroxyle] est compris entre 3 et 1000, de préférence compris entre 5 et 500, et mieux encore entre 5 et 120. L'expression « [nombre de fonctions acide + hydroxyle] »

Le rapport molaire [hydroxy acide]/[nombre de fonctions acides + hydroxyle] est compris entre 6 et 1000, de préférence compris entre 6 et 500, et mieux encore entre 6 et 120. L'expression « [nombre de fonctions acide + hydroxyle] » correspond en mole au nombre de fonctions réactives totales présentes :
- sur les monoglycérides, dans ce cas, il reste deux fonctions hydroxyles susceptibles de réagir par monoglycéride, ou sur les diglycérides, dans ce cas, il reste une fonction hydroxyle susceptible de réagir par diglycéride,
- sur les chaînes d'ester d'acide gras qui peuvent comporter un ou plusieurs groupements hydroxyles.

C'est en choisissant ce rapport molaire que l'on détermine la longueur moyenne des chaînes de polyacide qui seront greffées sur chaque fonction réactive du monoglycéride ou du diglycéride.

L'étape (b) est réalisée à une température comprise entre 100 et 220°C, de préférence entre 140 et 200°C.

Les hydroxy acides utilisés dans l'étape (b) peuvent être sous forme esters en vue de réaliser la réaction d'estérification-condensation selon un procédé de transestérification pour produire la chaîne de poly(hydroxy acide).

Enfin, selon une autre mode de réalisation de l'invention (Etape 2.b), il est possible de réaliser la condensation de l'hydroxy-acide de façon isolée et de faire ensuite réagir dans l'étape (b) le polyester formé sur le mélange de monoglycérides et de diglycérides préalablement préparé. Dans ce cas, on considère selon l'invention que l'on fait réagir un poly(hydroxy acide) déjà formé.

L'invention est illustrée par les exemples suivants.

### Exemple 1 : Préparation d'une résine lactique de monoléate de colza

### Etape a : Préparation d'un mélange riche en mono-oléate de colza

Dans un réacteur en verre équipé d'une agitation mécanique, on mélange 392,2 g d'huile de colza oléique, 67,6 g de glycérol et 3,3 g de soude à 99%. Le mélange est porté à 220°C et maintenu à cette température pendant 2 heures. La transformation totale des triglycérides est contrôlée par HPLC. En fin de réaction, le mélange est refroidi lentement avant d'être stocké.

### Etape b : préparation d'une résine naturelle par réaction de l'acide lactique sur un mélange riche en mono-oléate de colza

Dans un réacteur en verre équipé d'une agitation mécanique et d'un Dean-Stark, on mélange le produit obtenu lors de l'étape (a) (soit 463,1 g), avec 3101 g d'acide lactique à 80% et 28,8 g d'éthylhexanoate d'étain à 99%. Le mélange est porté à 150°C et maintenu à cette température pendant 9 heures. En fin de réaction, on obtient 2368 g de résine présentant les caractéristiques suivantes :
- Indice d'acide : 55
- Indice d'iode : 15
- Indice de péroxyde : 82
- Taux de cendre : 0,50.

Le produit préparé selon l'invention présente principalement la structure suivante.

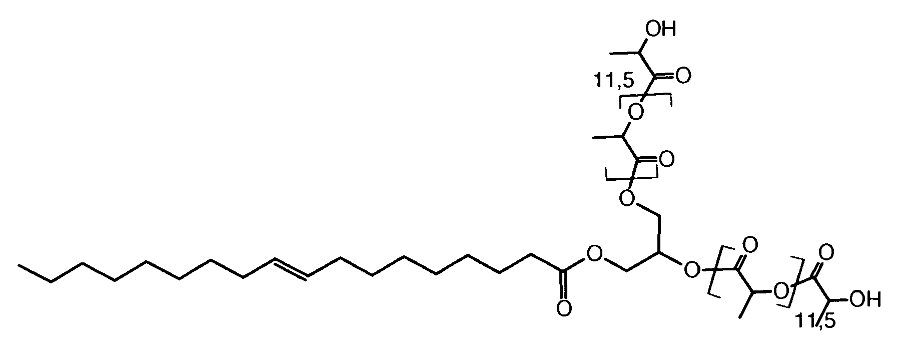

### Exemple II : Préparation d'une résine à base d'huile de ricin

Etape a : Préparation d'un mélange riche en mono-oléate de ricin

Dans un réacteur en verre équipé d'une agitation mécanique, on mélange 287,9 g d'huile de ricin, 42,2 g de glycérol et 2,1 g de soude à 99%. Le mélange est porté à 220°C et maintenu à cette température pendant 2 heures. La transformation totale des triglycérides est contrôlée par HPLC. En fin de réaction, le mélange est refroidi lentement avant d'être stocké.

### Etape b : Préparation d'une résine naturelle par réaction de l'acide lactique sur un mélange riche en mono-oléate de ricin

Dans un réacteur en verre équipé d'une agitation mécanique et d'un Dean-Stark, on mélange le produit obtenu dans l'étape a (soit 349,9 g), avec 3907,7 g d'acide lactique à 80% et 34,8 g d'éthylhexanoate d'étain à 99%. Le mélange est porté à 150°C et maintenu à cette température pendant 9 heures. En fin de réaction, on obtient 2773 g de résine présentant les caractéristiques suivantes :
- Indice d'acide : 68
- Indice d'iode : 9
- Indice de péroxyde : 97
- Taux de cendre : 0,43

## Revendications

1. Résine organique dérivée d'une huile ou d'une graisse d'origine naturelle comprenant des monoglycérides et/ou des diglycérides, estérifiés par un poly(hydroxy acide) répondant à la formule suivante : 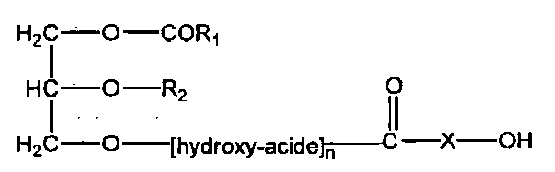 dans laquelle,
- R₁ est une chaîne hydrocarbonée aliphatique comportant 6 à 32 atomes de carbone, saturée ou insaturée, éventuellement substituée par des groupements alkyles ou hydroxyles,
- R₂ est un atome d'hydrogène, un groupe -COR₄ avec R₄ défini de la même façon que R₁ ou un groupe estérifié de poly(hydroxy acide),
- le groupement poly(hydroxy acide) correspondant à [hydroxy acide]ₙ-CO-X-OH est une chaîne linéaire ou ramifiée, obtenue par condensation de monomères d'hydroxy acide semblables ou différents,
- en fonction de la nature de l'hydroxy-acide, X = -CH₂, -CHR, R étant un groupe alkyle de 1 à 5 atomes de carbone et comportant 0 à 5 fonction(s) hydroxyle,
- n représente le nombre de motifs d'hydroxy acide identiques ou différents et est compris entre 10 et 2000.

2. Résine selon la revendication 1 **caractérisée en ce que** les hydroxy acides sont choisis parmi les acides α-hydroxylés tels que l'acide lactique, l'acide citrique, l'acide malique, l'acide tartarique, l'acide ascorbique et les acides β-hydroxylés tels que l'acide glycolique, l'acide salicylique, l'acide β-hydroxy butyrique, de préférence parmi l'acide lactique, l'acide citrique et l'acide malique.

3. Résine selon la revendication 1 ou 2 **caractérisée en ce que** le nombre de motifs d'hydroxy acide identiques ou différents est compris entre 10 et 1000, de préférence compris entre 10 et 500, et mieux encore 10 et 120.

4. Résine selon l'une quelconque des revendications précédentes **caractérisée en ce que** les monoglycérides et/ou diglycérides sont obtenus à partir d'huiles végétales ou animales choisies parmi les huiles de colza oléique et érucique, l'huile de lin, l'huile de tournesol, l'huile de ricin, l'huile de pourghère (*Jatropha curcas*), l'huile de soja, l'huile de palme, l'huile de palmiste, huile de coprah, l'huile de maïs, l'huile de coton, l'huile d'arachide, l'huile de son de riz, l'huile d'olive, l'huile de tung, les huiles de poisson, de micro et macro-algues, l'huile de suif et de tall-oil.

5. Résine selon l'une quelconque des revendications précédentes **caractérisée en ce** l'huile végétale comprend des acides gras comprenant entre 12 et 20 atomes de carbone et de préférence des acides gras en C18.

6. Résine selon l'une quelconque des revendications précédentes **caractérisée en ce qu'**elle est essentiellement d'origine naturelle, de préférence d'origine végétale.

7. Procédé de préparation d'une résine organique dérivée d'une huile ou d'une graisse d'origine naturelle naturelle comprenant des monoglycérides et/ou des diglycérides, estérifiés par un poly(hydroxy acide) répondant à la formule suivante : 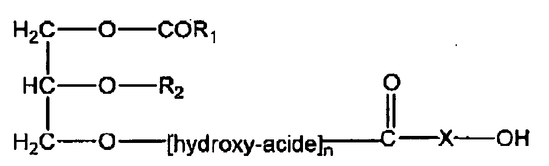 dans laquelle,
- R₁ est une chaîne hydrocarbonée aliphatique comportant 6 à 32 atomes de carbone, saturée ou insaturée, éventuellement substituée par des groupements alkyles ou hydroxyles,
- R₂ est un atome d'hydrogène, un groupe -COR₄ avec R₄ défini de la même façon que R₁ ou un groupe estérifié de poly(hydroxy acide),
- le groupement poly(hydroxy acide) correspondant à [hydroxy acide]ₙ-CO-X-OH est une chaîne linéaire ou ramifiée, obtenue par condensation de monomères d'hydroxy acide semblables ou différents,
- en fonction de la nature de l'hydroxy-acide, X = -CH₂, -CHR, R étant un groupe alkyle de 1 à 5 atomes de carbone et comportant 0 à 5 fonction(s) hydroxyle,
- n représente le nombre de motifs d'hydroxy acide identiques ou différents et est compris entre 5 et 2000,
**caractérisé en ce qu'**il comporte une étape (b) de mise en réaction :
- d'au moins un hydroxy acide ou un ester d'hydroxy acide en excès, ou d'un poly(hydroxy acide) déjà formé, et
- d'un mono et/ou un diglycéride.

8. Procédé de préparation selon la revendication 7 **caractérisée en ce** l'étape (b) est réalisée par réaction de 1 à 30 % en poids, de préférence 1 à 20 % en poids de monoglycéride et/ou de diglycéride avec 70 à 99 % en poids, de préférence 80 à 98 % en poids par rapport au poids total de la résine d'hydroxy acide ou d'un poly(hydroxy acide) déjà formé.

9. Procédé de préparation selon la revendication 7 ou 8 **caractérisé en ce que** l'on utilise dans l'étape (b) un catalyseur choisi dans le groupe constitué par les sels organiques d'étain, de fer, de zinc, d'aluminium, les acides minéraux ou organiques, les catalyseurs basiques, de préférence le catalyseur est l'éthylhexanoate d'étain (SnOct₂).

10. Procédé de préparation selon l'une quelconque des revendications 7 à 9 **caractérisé en ce que** l'étape (b) est réalisée à une température comprise entre 100 et 200°C, de préférence entre 140 et 160°C

11. Procédé de préparation selon l'une quelconque des revendications 7 à 10 **caractérisé en ce que** les hydroxy acides sont choisis parmi les acides α-hydroxylés tels que l'acide lactique, l'acide citrique, l'acide malique, l'acide tartarique, l'acide ascorbique et les acides β-hydroxylés tels que l'acide glycolique, l'acide salicylique, l'acide β-hydroxy butyrique, de préférence parmi l'acide lactique, l'acide citrique et l'acide malique.

12. Procédé de préparation selon l'une quelconque des revendications 7 à 11 **caractérisé en ce que** le nombre de motifs d'hydroxy acide identiques ou différents est compris entre 5 et 1000, de préférence compris entre 5 et 500, et mieux encore 5 et 120.

13. Procédé de préparation selon l'une quelconque des revendications 7 à 12 **caractérisé en ce que** les monoglycérides et/ou diglycérides sont obtenus à partir d'huiles végétales ou animales choisies parmi les huiles de colza oléique et érucique, l'huile de lin, l'huile de tournesol, l'huile de ricin, l'huile de pourghère *(Jatropha curcas),* l'huile de soja, l'huile de palme, l'huile de palmiste, huile de coprah, l'huile de maïs, l'huile de coton, l'huile d'arachide, l'huile de son de riz, l'huile d'olive, l'huile de tung, les huiles de poisson, de micro et macro-algues, l'huile de suif et de tall-oil.

14. Procédé de préparation selon l'une quelconque des revendications 7 à 9 **caractérisé en ce que** l'huile végétale comprend des acides gras comprenant entre 12 et 20 atomes de carbone et de préférence des acides gras en C18

15. Procédé de préparation selon l'une quelconque des revendications 7 à 14 **caractérisé en ce que** la résine est essentiellement d'origine naturelle, de préférence d'origine végétale.

## Claims

1. An organic resin derived from a naturally occurring oil or fat comprising monoglycerides and/or diglycerides, esterified with a poly(hydroxy acid) having the following formula: 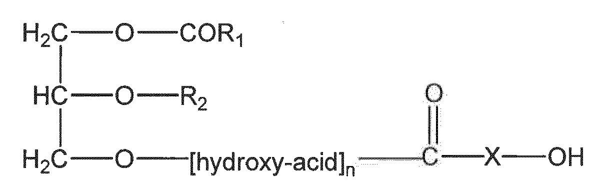 wherein,
- R₁ is a saturated or unsaturated, aliphatic hydrocarbon chain comprising from 6 to 32 carbon atoms, optionally substituted with alkyl or hydroxyl groups,
- R₂ is a hydrogen atom, a group -COR₄, where R₄ is defined according to the same definition as R₁ or a poly(hydroxy acid) esterified group,
- the poly(hydroxy acid) group corresponding to [hydroxy acid]ₙ-CO-X-OH is a linear or a branched chain, obtained y condensating hydroxy monomers that are the same or different,
- depending on the nature of the hydroxy acid, X = -CH₂, -CHR, where R is an alkyl group having from 1 to 5 carbon and comprising from 0 to 5 hydroxyl function(s),
- n corresponds to the number of hydroxy acid units that are the same or different and ranges from 10 to 2000.

2. A resin according to claim 1, wherein the hydroxy acids are selected from α-hydroxylated acids such as lactic acid, citric acid, malic acid, tartaric acid, ascorbic and β-hydroxylated acids such as glycolic acid, salicylic acid, β-hydroxy butyric acid, preferably from lactic acid, citric acid and malic acid.

3. A resin according to claim 1 or 2, wherein the number of hydroxy acid units that are the same or different does range from 10 to 1000, preferably from 10 to 500, and more preferably from 10 to 120.

4. A resin according to anyone of preceding claims, wherein the monoglycerides and/or diglycerides are from vegetable or animal oils selected from oleic and erucic rapeseed oils, linseed oil, sunflower seed oil, castor oil, *Jatropha curcas* oil, sojabean oil, palm oil, palm kernel oil, coconut oil, corn oil, cottons oil, groundnut oil, rice bran oil, olive oil, China wood oil, fish oils, micro- and macro-alga oils, tallow oil and tall oil.

5. A resin according to anyone of preceding claims, wherein the vegetable oil comprises fatty acids having from 12 to 20 carbon atoms and preferably fatty acids having 18 carbon atoms.

6. A resin according to anyone of preceding claims, which is of substantially natural origin, preferably of vegetable origin.

7. A method for preparing an organic resin derived from a naturally occurring oil or fat comprising monoglycerides and/or diglycerides, esterified with a poly(hydroxy acid) having the following formula: 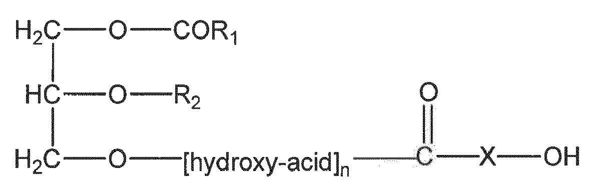 wherein,
- R₁ is a saturated or unsaturated, aliphatic hydrocarbon chain comprising from 6 to 32 carbon atoms, optionally substituted with alkyl or hydroxyl groups,
- R₂ is a hydrogen atom, a group -COR₄, where R₄ is defined according to the same definition as R₁ or a poly(hydroxy acid) esterified group,
- the poly(hydroxy acid) group corresponding to [hydroxy acid]ₙ-CO-X-OH is a linear or a branched chain, obtained by condensating hydroxy acid monomers that are the same or different,
- depending on the nature of the hydroxy acid, X = -CH₂, -CHR, where R is an alkyl group having from 1 to 5 carbon atoms and comprising from 0 to 5 hydroxyl function(s),
- n corresponds to the number of hydroxy acid units that are the same or different and ranges from 5 to 2000,
**characterized in that** it comprises a step (b) of reacting:
- at least one hydroxy acid or one hydroxy acid ester in excess, or one already formed poly(hydroxy acid), with
- a mono and/or a diglyceride.

8. A preparation method according to claim 7, wherein step (b) is carried out by reacting from 1 to 30% by weight, preferably from 1 to 20% by weight of the monoglyceride and/or diglyceride with from 70 to 99% by weight, preferably with from 80 to 98% by weight as related to the total weight of the hydroxy acid resin or the already formed poly(hydroxy acid).

9. A preparation method according to claim 7 or 8, wherein a catalyst is used in step (b) selected in the group consisting of tin, iron, zinc, and aluminium organic salts, inorganic or organic acids, basic catalysts, preferably the catalyst is tin ethylhexanoate (SnOct₂).

10. A preparation method according to anyone of claims 7 to 9, wherein step (b) is conducted at a temperature ranging from 100 and 200°C, preferably from 140 to 160°C.

11. A preparation method according to anyone of claims 7 to 10, wherein the hydroxy acids are selected from α-hydroxylated acids such as lactic acid, citric acid, malic acid, tartaric acid, ascorbic acid and β-hydroxylated acids such as glycolic acid, salicylic acid, β-hydroxy butyric acid, preferably from lactic acid, citric acid and malic acid.

12. A preparation method according to anyone of claims 7 to 11, wherein the number of hydroxy acid units that are the same or different does range from 5 to 1000, preferably from 5 to 500, and more preferably from 5 to 120.

13. A preparation method according to anyone of claims 7 to 12, wherein the monoglycerides and/or diglycerides are derived from vegetable or animal oils selected from oleic and erucic rapeseed oils, linseed oil, sunflower seed oil, castor oil, *Jatropha curcas* oil, sojabean oil, palm oil, palm kernel oil, coconut oil, corn oil, cottonseed oil, groundnut oil, rice bran oil, olive oil, China wood oil, fish oils, micro- and macro-alga oils, tallow oil and tall oil.

14. A preparation method according to anyone of claims 7 to 9, wherein the vegetable oil comprises fatty acids having from 12 to 20 carbon atoms and preferably fatty acids having 18 carbon atoms.

15. A preparation method according to anyone of claims 7 to 14, wherein the resin is of substantially natural origin, preferably of vegetable origin.

## Patentansprüche

1. Organisches Harz, abgeleitet von einem Öl oder Fett natürlichen Ursprungs, das Monoglyceride und/oder Diglyceride umfasst, die durch eine Polyhydroxysäure verestert sind und der folgenden Formel entsprechen: 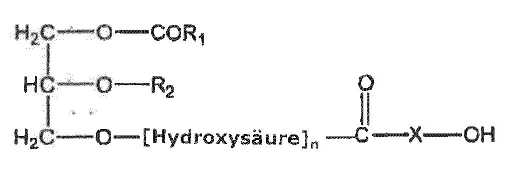 wobei
- R₁ eine gesättigte oder ungesättigte aliphatische Kohlenwasserstoffkette mit 6 bis 32 Kohlenstoffatomen ist, die gegebenenfalls mit Alkyl- oder Hydroxygruppen substituiert ist;
- R₂ ein Wasserstoffatom, eine Gruppe -COR₄, wobei R₄ wie R₁ definiert ist, oder eine veresterte Polyhydroxysäuregruppe ist;
- die Polyhydroxysäuregruppe, die [Hydraxysäure]ₙ-CO-X-OH entspricht, eine lineare oder verzweigte Kette ist, die durch Kondensation von gleichen oder verschiedenen Hydroxysäuremonomeren erhalten wird;
- je nach Art der Hydroxysäure X = -CH₂ oder -CHR ist, wobei R eine Alkylgruppe mit 1 bis 5 Kohlenstoffatomen ist, die 0 bis 5 Hydroxyfunktionen umfasst;
- n der Anzahl der gleichen oder verschiedenen ydroxysäureeinheiten entspricht und zwischen 10 und 2000 liegt.

2. Harz gemäß Anspruch 1, **dadurch gekennzeichnet, dass** die Hydroxysäuren aus α-Hydroxysäuren, wie Milchsäure, Zitronensäure, Äpfelsäure, Weinsäure, Ascorbinsäure, und β-Hydroxysäuren, wie Glycolsäure, Salicylsäure, β-Hydroxybuttersäure, und vorzugsweise aus Milchsäure, Zitronensäure und Äpfelsäure ausgewählt sind.

3. Harz gemäß Anspruch 1 oder 2, **dadurch gekennzeichnet, dass** die Anzahl der gleichen oder verschiedenen Hydroxysäureeinheiten zwischen 10 und 1000, vorzugsweise zwischen 10 und 500 und besonders bevorzugt zwischen 10 und 120 liegt.

4. Harz gemäß einem der vorstehenden Ansprüche, **dadurch gekennzeichnet, dass** die Monoglyceride und/oder Diglyceride ausgehend von pflanzlichen oder tierischen Ölen erhalten sind, die aus ölsäurereichem und erucasäurereichem Rapsöl, Leinöl, Sonnenblumenöl, Ricinusöl, Jatrophaöl, Sojaöl, Palmöl, Palmkernöl, Kokosöl, Maisöl, Baumwollöl, Erdnussöl, Reiskleieöl, Olivenöl, Tungöl, Fischöle, Mikro- und Makroalgenöl, Talgöl und Tallöl ausgewählt sind.

5. Harz gemäß einem der vorstehenden Ansprüche, **dadurch gekennzeichnet, dass** das Pflanzenöl Fettsäuren mit 12 bis 20 Kohlenstoffatomen und vorzugsweise C₁₈-Fettsäuren umfasst.

6. Harz gemäß einem der vorstehenden Ansprüche, **dadurch gekennzeichnet, dass** es im Wesentlichen natürlichen Ursprungs, vorzugsweise pflanzlichen Ursprungs, ist.

7. Verfahren zur Herstellung eines organischen Harzes, das von einem Öl oder Fett natürlichen Ursprungs abgeleitet ist, das Monoglyceride und/oder Diglyceride umfasst, die durch eine Polyhydroxysäure verestert sind und der folgenden Formel entsprechen: 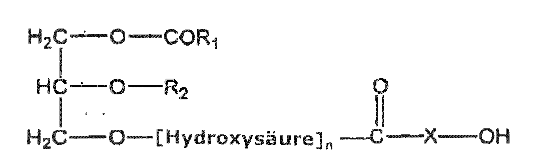 wobei
- R₁ eine gesättigte oder ungesättigte aliphatische Kohlenwasserstoffkette mit 6 bis 32 Kohlenstoffatomen ist, die gegebenenfalls mit Alkyl- oder Hydroxygruppen substituiert ist;
- R₂ ein Wasserstoffatom, eine Gruppe -COR₄, wobei R₄ wie R₁ definiert ist, oder eine veresterte Polyhydroxysäuregruppe ist;
- die Polyhydroxysäuregruppe, die [Hydroxysäure]ₙ-CO-X-OH entspricht, eine lineare oder verzweigte Kette ist, die durch Kondensation von gleichen oder verschiedenen Hydroxysäuremonomeren erhalten wird;
- je nach der Hydroxysäure X = -CH₂ oder -CHR ist, wobei R eine Alkylgruppe mit 1 bis 5 Kohlenstoffatomen ist, die 0 bis 5 Hydroxyfunktionen umfasst;
- n der Anzahl der gleichen oder verschiedenen ydroxysäureeinheiten entspricht und zwischen 5 und 2000 liegt;
**dadurch gekennzeichnet, dass** es einen Schritt (b) der Umsetzung
- wenigstens einer Hydroxysäure oder eines Hydroxysäureesters im Überschuss oder einer bereits gebildeten Polyhydroxysäure und
- eines Mono- und/oder Diglycerids
umfasst.

8. Herstellungsverfahren gemäß Anspruch 7, **dadurch gekennzeichnet, dass** Schritt (b) durch Reaktion von 1 bis 30 Gew.-%, vorzugsweise 1 bis 20 Gew.-%, Monoglycerid und/oder Diglycerid mit 70 bis 99 Gew.-%, vorzugsweise 80 bis 98 Gew.-%, bezogen auf das Gesamtgewicht des Harzes, einer Hydroxysäure oder einer bereits gebildeten Polyhydroxysäure durchgeführt wird.

9. Herstellungsverfahren gemäß Anspruch 7 oder 8, **dadurch gekennzeichnet, dass** in Schritt (b) ein Katalysator verwendet wird, der aus der Gruppe ausgewählt ist, die aus organischen Salzen von Zinn, Eisen, Zink, Aluminium, Mineralsäuren oder organischen Säuren, basischen Katalysatoren besteht, wobei es sich bei dem Katalysator vorzugsweise um Zinnethylhexanoat (SnOCt₂) handelt.

10. Herstellungsverfahren gemäß einem der Ansprüche 7 bis 9, **dadurch gekennzeichnet, dass** Schritt (b) bei einer Temperatur von 100 bis 200°C, vorzugsweise 140 bis 160°C, durchgeführt wird.

11. Herstellungsverfahren gemäß einem der Ansprüche 7 bis 10, **dadurch gekennzeichnet, dass** die Hydroxysäuren aus α-Hydroxysäuren, wie Milchsäure, Zitronensäure, Äpfelsäure, Weinsäure, Ascorbinsäure, und β-Hydroxysäuren, wie Glycolsäure, Salicylsäure, β-Hydroxybuttersäure, und vorzugsweise aus Milchsäure, Zitronensäure und Äpfelsäure ausgewählt sind.

12. Herstellungsverfahren gemäß einem der Ansprüche 7 bis 11, **dadurch gekennzeichnet, dass** die Anzahl der gleichen oder verschiedenen Hydroxysäureeinheiten zwischen 5 und 1000, vorzugsweise zwischen 5 und 500 und besonders bevorzugt zwischen 5 und 120 liegt.

13. Herstellungsverfahren gemäß einem der Ansprüche 7 bis 12, **dadurch gekennzeichnet**, die Monoglyceride und/oder Diglyceride ausgehend von pflanzlichen oder tierischen Ölen erhalten sind, die aus ölsäurereichem und erucasäurereichem Rapsöl, Leinöl, Sonnenblumenöl, Ricinusöl, Jatrophaöl, Sojaöl, Palmöl, Palmkernöl, Kokosöl, Maisöl, Baumwollöl, Erdnussöl, Reiskleieöl, Olivenöl, Tungöl, Fischöle, Mikro- und Makroalgenöl, Talgöl und Tallöl ausgewählt sind.

14. Herstellungsverfahren gemäß einem der Ansprüche 7 bis 9, **dadurch gekennzeichnet, dass** das Pflanzenöl Fettsäuren mit 12 bis 20 Kohlenstoffatomen und vorzugsweise C₁₈-Fettsäuren umfasst.

15. Herstellungsverfahren gemäß einem der Ansprüche 7 bis 14, **dadurch gekennzeichnet, dass** das Harz im Wesentlichen natürlichen Ursprungs, vorzugsweise pflanzlichen Ursprungs, ist.
